Europäisches Patentamt

European Patent Office

Office européen des brevets

(11) Publication number: **0 232 171 B1**

# EUROPEAN PATENT SPECIFICATION

(45) Date of publication of patent specification: **25.11.92**   (51) Int. Cl.⁵: **B32B 27/08**, A61J 1/00

(21) Application number: **87301020.1**

(22) Date of filing: **05.02.87**

(54) **Sterilizable multi-layer plastic materials for medical containers and the like.**

(30) Priority: **07.02.86 US 827847**

(43) Date of publication of application:
**12.08.87 Bulletin 87/33**

(45) Publication of the grant of the patent:
**25.11.92 Bulletin 92/48**

(84) Designated Contracting States:
**BE DE FR GB**

(56) References cited:
EP-A- 0 083 778      EP-A- 0 142 315
EP-A- 0 168 755      EP-A- 0 216 639
US-A- 4 465 487      US-A- 4 497 857

(73) Proprietor: **BAXTER INTERNATIONAL INC. (a Delaware corporation)**
**One Baxter Parkway**
**Deerfield Illinois 60015(US)**

(72) Inventor: **Measells, Paul E.**
**1301 Wyndham Drive 205**
**Palatine Illinois 60067(US)**
Inventor: **Laurin, Dean G.**
**564 Applegate Lane**
**Lake Zurich Illinois 60047(US)**
Inventor: **Johnston, William D.**
**801 Dannet**
**Buffalo Grove Illinois 60089(US)**
Inventor: **Czuba, Leonard F.**
**1105 East Adams**
**Lombard Illinois 60148(US)**
Inventor: **Kwong, Peter C.**
**1112 Sunset Road**
**McHenry Illinois 60050(US)**

(74) Representative: **MacGregor, Gordon et al**
**ERIC POTTER & CLARKSON St. Mary's Court**
**St. Mary's Gate**
**Nottingham, NG1 1LE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

# Description

## FIELD OF THE INVENTION

The invention pertains to multi-layer plastic materials and containers made therefrom. More particularly, the invention pertains to sterilizable multi-layer plastic materials and containers suitable for use in the medical field.

## BACKGROUND OF THE INVENTION

Various forms of multi-layer plastic films are well-known in the art. For example, Schirmer United States No. 3,832,270, entitled "Heat Shrinkable, Oriented Laminated Plastic Film", discloses a multi-layer package wrapping film. The film of the Schirmer patent is a multi-layer laminated structure with a layer of enthylene-vinyl acetate bonded to a layer of polyethylene. Prior to the lamination step, the layer of ethylene-vinyl acetate is irradiated to effect cross-linking.

Further, Nakamura et al United States Patent No. 4,465,487, entitled "Container for Medical Use", discloses a plastic container formed of ethylene-vinyl acetate. The collapsible container is formed of a single layer sheet material. This material has a preferred thickness in a range of 0.010 - 0.015 inch (0.25 - 0.38 mm). The container is first heat sealed at its edges to provide fluid resistant seals. Then, the container is irradiated to provide desired cross-linking in the material to provide for heat resistance during autoclaving. Also in the Nakamura et al patent, a port is affixed to the body of the container after cross-linking has taken place, using non-cross-linked meltable plastic as an adhesive.

Poly(ethylene-vinyl acetate) materials, often referred to as EVA materials, are sufficiently flexible to form containers usable in the medical field. One advantage of EVA materials is that they do not require plasticizers, as do polyvinyl chloride materials. As a result, the quantity of leachable materials or extractable components can be essentially eliminated from the plastic film. Further, prior to cross-linking, the EVA material is easily heat-sealable employing, for example, dielectric heating methods using radio frequency voltages between metal heat sealing discs.

However, known conventional containers made from EVA materials have certain distinct disadvantages. For example, when subjected to pasteurizing or autoclaving temperatures on the order of 110°C or more, the EVA materials tend to soften, distort or melt. The softening effect can be inhibited by high energy irradiation of the EVA material to provide the necessary and desirable cross-linking, thus improving the heat-resistant characteristics of the EVA material. However, all heat-sealing of the material to provide fluid resistant seals must be performed prior to the irradiation step.

In addition, known conventional EVA containers tend to have wall thicknesses on the order of 0.015 inch (0.38 mm). This wall thickness has been conventionally utilized in connection with radio frequency, dielectric heat sealing, because it results in acceptable heat sealed joints with minimal electric arcing, given present techniques and manufacturing equipment. Additionally, such wall thicknesses provide an acceptable level of strength.

However, with conventional single layer EVA films, irradiation doses on the order of 15 to 40 megarads are required to produce the degree of cross-linking necessary to withstand sterilization temperatures. Such high irradiation dosage levels, coupled with the relatively large amounts of EVA materials present, cause undesirably high amounts of acetic acids or other extractable materials. The acetic acid produced is undesirable because it can be absorbed by the contents of the container.

Therefore, when using EVA materials in the medical field, it is desirable to use lower irradiation doses and lesser amounts of EVA materials, thereby producing lesser amounts of acetic acid and other extractable byproducts.

Further, known conventional EVA materials suffer from a further disadvantage in that the extrusion process must be carefully designed and controlled. A lack of control during the extrusion process can result in internal stresses, or "frozen-in" stresses, in the material. At room temperature these stresses may be of minimum consequence. However, when containers formed of internally stressed materials are heated within a sterilizing unit, such as a steam autoclave, the internal stresses can lead to excessive shrinkage or distortion of the heated container. In extreme cases, these internal stresses can even cause the containers to rupture.

In addition, known conventional containers formed of EVA materials have to be positioned carefully during sterilization. Adjacent containers in the sterilizing unit tend to adhere or stick to one another due the melting and recrystallizing of the EVA material, even though it may have been sufficiently cross-linked to prevent distortion.

In additional to all of the above-stated disadvantages, containers made of flexible plastics which are radiation crosslinkable, as are EVA materials, are also highly permeable to water, oxygen, and carbon dioxide. Therefore, such containers simply cannot be used for storing aqueous solutions without employing a barrier overwrap. This only adds to the overall expense of the container, while at the same time detracting from user convenience.

One objective of this invention is to provide a method of making medical grade, transparent, flexible plastic container which is readily fillable and drainable, which does not incorporate materials having plasticizers, and which minimizes migration of materials, such as acetic acid, into the contents of the container. The container may be as strong as prior art containers, being able to withstand a six foot drop test when filled, and may be less subject to distorting or rupturing, due to internal stresses, when heat sterilized and will exhibit little or no container-to-container tackiness during or after heat sterilization. Barrier materials may be integrally included, eliminating the need for a separate barrier overwrap, thereby adding to the overall economies and the convenience of use.

The container may, in addition to the above-stated advantages, be formed using dielectric radio frequency heat sealing methods, thereby providing stronger fluid resistant seals than are possible with impulse or hot-bar heat sealing methods.

The precharacterising part of Claim 1 is based on US-A-4497857. This document discloses a method of fabricating a heat-sterilisable substance-retaining container, comprising forming first and second film members, each having at least first and second integral layers, the first layer being heat-sealable, positioning the first and second film members in overlapping relationship with corresponding first layers of the film members being between the second layers to form inner layers of the container; positioning a port with an outer cylindrical surface between regions of the film members, and melting a peripheral region of the overlapping, first layers using heat-sealing elements, so as to form the container by forming a substance-retaining seal between the first layers thereby securing said port, the second layers staying cooler to prevent thinning or cut-through by the heat sealing elements, filling the container, and sterilising the filled container by exposure to heat, the first layers being distortable at the sterilising temperature. An inner barrier layer is provided.

The present invention is characterised in that:-

the material of the first layers is selected to be free, or essentially free, of leachable plasticisers and to be cross-linkable,

the material of the second layers is selected not to be cross-linkable, as compared with the first layers, and to have a higher melting temperature so as not to be distortable at the sterilising temperature,

the material of the outer surface of the port is selected to be sealed to the first layers during the melting step and to be cross-linkable, and

the container is irradiated, prior to the filling and sterilising steps, to cross-link the first layers and the outer surface of the port and provide resistance of the first layers to distortion at the sterilising temperature.

The second layer provides enhanced physical stability to the overall film and articles made from the film, both during and after sterilization. The second layer also does not exhibit tackiness during sterilization. Preferably, the second layer also provides a barrier to water vapor to prevent evaporation through the film.

The film can be formed either by coextrusion or by lamination.

In a preferred embodiment, the first layer comprises poly(ethylene vinyl acetate), i.e., EVA. In this arrangement, the second layer preferably comprises a high-density linear polyethylene or copolymers consisting substantially of linear polyethylene.

More particularly, the multi-layer plastic material preferably can be formed of an EVA layer having from 10 to 50 weight percent of vinyl acetate units, most preferable 10-35% by weight. The high-density polyethylene layer may also be formed of substantially ethylene copolymers and their blends with lesser amounts of other materials. In addition, substantially crystalline polyolefin may be used, such as polymers of substantially propylene, 4-methyl pentene-1, butene-1 or the like.

Preferably, the multi-layer material will have a thickness on the order of 0.007 inch (0.18 mm) thick for the EVA material and 0.003 inch (0.08 mm) thick for the high-density polyethylene material, resulting in an overall thickness of only .010 inch (0.25 mm).

A container can be formed by positioning two sheets of the multi-layer film adjacent one another with the EVA layers facing each other. The seams of the container can be sealed by means of radio frequency (RF) energy or conductive welding applied to overlapping regions of EVA material. Radio frequency electrodes can be positioned adjacent to the exterior, high-density, polyethylene layers. The energy induced by the RF field causes the EVA layers in the region between the electrodes to heat, melt and fuse together. The polyethylene layers in the same region do not generate heat as the EVA layers do. Hence, the polyethylene layers stay cooler and resist flowing out from between the electrodes. In addition, the higher melting temperature of the second layer, when compared to the first layer, helps prevent excessive thinning or cut-through by conductive heat sealing elements.

The exterior two layers of polyethylene thus provide physical and structural stability to the seam while the EVA material is in a liquid state and can readily flow. Further, the non-melted polyethylene layers do not flow from between the RF electrodes. This prevents the electrodes from coming too close to each other, with resultant arcing, which results in inadequate and non-uniform seals. As a result of this interaction between the high-density exterior polyethylene layers, a much more consistent heat seal is created.

The inwardly oriented EVA layers, when heat sealed together in the manner just described, form a non-leaking container. In this container, the contents are in contact only with surfaces of the EVA layers, which are free of plasticizers. In this configuration, the higher strength, higher temperature resistant, high-density polyethylene exterior layers, in combination with the cross-linked EVA-layers, provide a container as strong as, but more temperature resistant than containers made using single layer EVA films.

Because the EVA layer in the multi-layer material is relatively thin and supported by the second polyethylene layer, the composite can be cross-linked to the desired degree by exposure to a selected, relatively low, level of irradiation, on the order of 5 to 10 megarads. Because of this lower radiation dosage, fewer extractable by-products, such as acetic acid or the like, are produced by the material during the irradiation step. Subsequently, the container can be filled with a selected fluid or solid and the entire unit can be sterilized without developing leaks.

Sterilization can be by means of an autoclave, as is conventional. Alternately, instead of steam sterilization, dry heat or radiation sterilization may also be used to sterilize both the container and its contents.

When the material is coextruded, any internal stresses that may be present in the EVA layer of the coextruded material will be partly supported by the high-density polyethylene layer during the heat sterilization process. As a result, there is less relaxation, shrinkage or distortion during sterilization.

Preferably, the port is formed of a tubular member of multi-layer material. In this configuration, the EVA layer is positioned on an exterior surface of the port. The high-density polyethylene is positioned on an interior peripheral surface of the port. The port is positioned between unsealed, overlapping regions of the body portion of the container. The exterior EVA layer of the port is thus located adjacent the interior EVA layers of the body section.

The body portions of the container and the adjacent port may be heat sealed together using RF energy. A portion of the exterior EVA layer of the port member will be heat sealed to adjacent regions of EVA material of the body section. The container and port can then be subjected to irradiation, preferably on the order of 7.5 megarads, to produce the desired cross-linking. The EVA layer of the port will be subjected to cross-linking along with the EVA layer of the body portion.

After cross-linking, the container can be filled through the port. The port can then be sealed with a pierceable membrane or the like. The membrane can be formed of high-density polyethylene or other plastics which can withstand the temperatures of sterilization. The membrane can thus be heat sealed to the interior high density polyethylene surface of the port.

Finally, the sealed container and contents can be subjected to sterilization. Sterilization may be achieved using steam sterilization in an autoclave. Dry heat or radiation sterilization may also be used.

In addition to the above-noted benefits, the sealed container made according to the present invention resists sterilization temperatures in excess of 110°C without the heat seals failing. Further, the exterior high-density polyethylene surfaces do not exhibit undesirable tacking and do not adhere to adjacent containers while undergoing sterilization.

The container made in accordance with the present invention is especially adapted for storing materials that cannot be irradiated but which can be autoclaved. These include intravenous solutions such as dextrose, lipids, proteins, amino acids, or various drugs.

It will also be understood that materials having more than two layers can also be incorporated into containers in accordance with the present invention.

A third layer can be incorporated between the inner layer of EVA material and the outer layer of polyethylene, or within the EVA layer itself. When positioned between the EVA layer and the polyethylene layer, the third layer could be formed of poly(ethylene vinyl alcohol), referred to as EVAL. When positioned within the EVA layer, the third layer could be formed of either EVAL or polyvinylidine chloride, referred to as PVDC. In either embodiment, the third layer functions as a gas barrier layer. It can have a thickness in a range of 0.0001 to 0.005 inch (0.002 to 0.127 mm), preferably in a range of 0.0005 to 0.002 inch (0.013 to 0.051 mm).

Numerous other advantages and features of the present invention will become readily apparent from the following detailed description of the invention and the embodiments thereof, from the claims and from the accompanying drawings in which the details of the invention are fully and completely disclosed as a part of this specification.

DESCRIPTION OF THE DRAWINGS

Figure 1 is a view in perspective of a two layer section of the multi-layer film material which embodies the features of the invention;
Figure 2 is a sectional view of a portion of the multi-layer film of Figure 1;

Figure 3 illustrates a first step in forming a pouch of multi-layer film in accordance with the present invention;

Figure 4 illustrates schematically heat sealing regions of the pouch of multi-layer film of Figure 3;

Figure 5 is an end view, partly broken away, of the pouch of Figure 4;

Figure 6 illustrates an initial step in forming a sealed container of multi-layer film in accordance with the present invention;

Figure 7 is an end view of a tubular port usable with the container of Figure 6;

Figure 8 illustrates schematically heat sealing regions of the container of Figure 6;

Figure 9 is a view in section, taken along line 9-9 of Figure 8 illustrating the heat seal between the port and the body portion of the container;

Figure 10 is a sectional view of one embodiment of a three layer film material;

Figure 11 is a perspective view of the orientation of two sheets of the three layer films shown in Fig. 10 in the formation of the container;

Figure 12 is a sectional view of another embodiment of a four layer film material;

Figure 13 is a sectional view of a three layer film material;

Figure 14 is a sectional view of a multiple layer composite film material; and

Figure 15 is a sectional view of another multiple layer composite film material.

## DESCRIPTION OF THE PREFERRED EMBODIMENTS

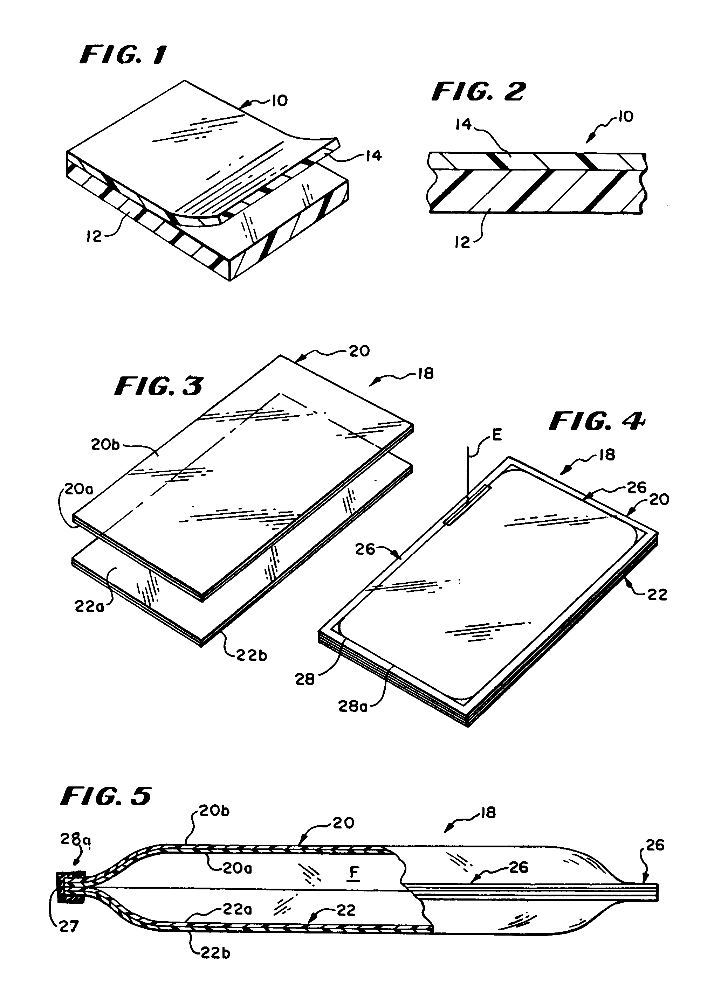

With respect to the Figures, Figure 1 illustrates a section of multi-layer film 10 having a first layer 12 and a second layer 14. The first layer 12 is composed of a cross-linkable material which is essentially free of leachable, or extractable, plasticizers. In the preferred and illustrated embodiment, the first layer 12 is poly(ethylene-vinyl acetate), referred to as EVA. The EVA layer 12 can be of material having 10 to 50 weight percent of vinyl acetate units and most preferably in a range of 10-35 weight percent, such as 18 weight percent of vinyl acetate units.

The second layer 14 is composed of a material which is, when compared to the material of the first layer 12, essentially non-crosslinkable. In addition, the material of the second layer 14 has a higher melting temperature than the material of the first layer 12. In the preferred and illustrated embodiment, the second layer 14 is high-density polyethylene. The polyethylene layer 14 can have a density of 0.94 to 0.965 g/cm³. Preferably, this density will be in a range of 0.95 to 0.955 g/cm³. The polyethylene layer 14 may include minor amounts of toughening and clarifying agents, such as rubbers and crystal-nucleating compounds.

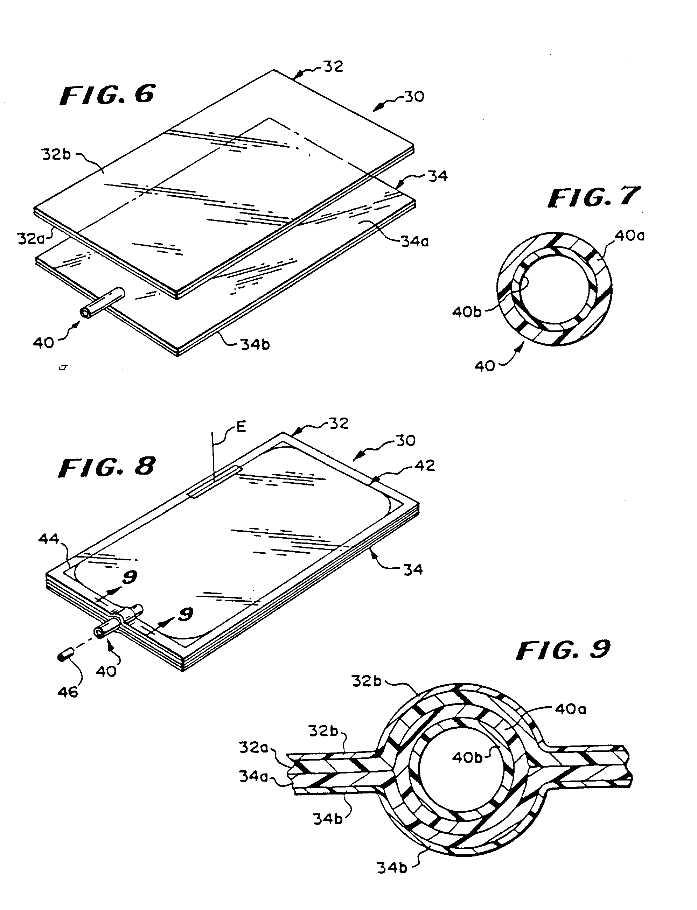

Figure 2 is a view in section of the film 10 illustrating the layer 12 bonded to the layer 14.

The EVA layer 12 can be formed with a thickness in a range of 0.005-0.010 inch (0.13 to 0.25 mm). The EVA layer 12 begins to soften at temperatures of approximately 60°C, and its crystalline portions begin to melt at temperatures of approximately 85°C.

The second layer 14 of high-density polyethylene can be formed with a thickness in a range of 0.002-0.005 inch (0.05-0.13 mm). The polyethylene layer 14 begins to soften at temperatures of approximately 125°C, and its crystalline portions begin to melt at temperatures in the range of approximately 135°C to 140°C. Preferably, the EVA layer 12 has a thickness substantially on the order of 0.007 inch (0.18 mm) and the high-density, polyethylene layer 14 has a thickness on the order 0.003 inch (0.076 mm). The resultant multi-layer material 10, then, has a preferred total thickness on the order of only 0.010 inch (0.25 mm).

The multi-layer material 10 can be formed using conventional coextrusion processes which are well-known in the art. It will be understood that alternate forms of bonding, such as those achieved by lamination, could also be used.

Multi-layer sheet materials, such as the film 10, exhibit desirable heat sealing characteristics and also exhibit low water vapor transmission characteristics. The fact that the material 10 has a thickness of only 0.010 inch (0.25 mm), as opposed to a thickness on the order of 0.015 inch (0.38 mm) as has often been used in the prior art, is also desirable because less material is needed, thereby costing less as well as having lesser amounts of leachable byproducts, contaminants, or additives.

The film 10 can then be assembled into a pouch or sealed container utilizing conventional conductive or radio frequency sealing machinery and techniques. Using the film 10, the layer 12, which contains no plasticizers, is the only material in the pouch or container in contact with the contents.

Figure 3 illustrates a first step in forming a fluid retaining pouch 18 utilizing the film 10. As illustrated in Figure 3, sheets 20 and 22 of film 10 are positioned adjacent one another, with EVA layers 20a and 22a, respectively, of each of the sheets 20 and 22, inwardly oriented. Layers 20b and 22b, corresponding to the high-density polyethylene layer 14, are outwardly oriented.

The sheets 20 and 22 may then be heat sealed together using RF energy, as illustrated schematically in Figure 4. In the heat sealing process, a plurality of overlapping, adjacent regions, such as the regions 26, of the EVA layers 20a and 22a are subjected to radio frequency energy as is well-known in the art. As a result, the adjacent EVA material positioned between the electrodes, such as the partial electrode E shown schematically in Figure 4, melts and fuses together forming a fluid retaining heat seal. The heat seal is indicated by solid lines 28.

A region 28a is not heat sealed together, thereby providing an opening through which the desired contents of the pouch 18 can be later introduced.

It will be understood that the heat seal 28 could be formed in one step with appropriately shaped electrodes. The particular electrodes or other equipment used to form the heat seals are not a limitation of the present invention.

The exterior high-density polyethylene layers, corresponding to the layers 20b and 22b, when positioned between the partial electrodes E, provide physical stability to the heated and molten EVA material during this process. Flow of the material away from the region of the partial electrodes E is minimized because the polyethylene layers resist flowing more than the EVA layers. As a result, much more consistent heat seals are achieved. In addition, the high-density polyethylene layer 14 tends to help reduce electrical arcing problems.

It should be noted that the heat seal 28, as illustrated in Figure 5, is formed between the molten EVA layers, such as the layers 20a and 22a. In the region of the heat seal 28, the EVA layers 20a and 22a have been fused into a single layer. The exterior layers 20b and 22b continue to retain their separate identity.

The pouch 18 can be irradiated, prior to being filled, with a dose on the order of 5 to 10 megarads, so as to produce the desired cross-linking in the fused EVA layers 20a and 22a and in the heat seal 28. The preferred dose of irradiation is on the order of 7.5 megarads.

It has been found that the pouch 18 as described, when subjected to the above-noted doses of irradiation, exhibits essentially the same impact resistance when dropped and the same heat resistance when sterilized as do bags which are formed with single layer EVA materials on the order of 0.015 inch (0.38 mm) thick. Such bags, as is well-known, require doses of irradiation on the order of 15 to 40 megarads to produce the desired cross-linking to withstand sterilization temperatures.

The higher doses of irradiation associated with conventional single layer EVA films result in a higher level of acetic acid and other extractables being formed during the manufacturing process. These materials are undesirable because they may contaminate the contents of the pouch 18.

The pouch 18 of Figures 4 and 5 can be subsequently filled through the open region 28a with an autoclavable substance, which is designated F in Fig. 5. After it has been filled, the open region 28a of the pouch 18 can be closed by a mechanical clip 27 or the like. The filled pouch 18 can now be sterilized in a steam autoclave. Alternately, dry heat sterilization can be used.

Figure 6 illustrates an initial step in the formation of a sealed container 30 formed of the multilayer film 10. The container 30 includes first and second body members 32 and 34. The members 32 and 34 each have inwardly oriented surfaces 32a and 34a, corresponding to the EVA layer 12 of the film 10.

The container 30 includes a port 40 formed from a coextruded, tubular member formed of the same material as the members 32 and 34. As shown in Figure 7, the port 40 has an outer EVA layer 40a and an inner, high-density polyethylene layer 40b. The EVA layer 40a forms an exterior surface and the high-density polyethylene layer 40b, forms an interior surface. The port 40 can be positioned between the sheet members 32 and 34 as shown in Figure 6. When such positioning occurs, the exterior EVA layer 40a is positioned adjacent the interior EVA layers 32a and 34a of the members 32 and 34.

Alternately (not shown), the port 40 may comprise a single EVA layer or multiple layers of various other materials with an outer layer of EVA and, optionally, an inner layer of EVA as well.

As illustrated in Figure 8, partial electrodes E, illustrated in schematic form, can be used to apply radio frequency energy to the overlapping regions 42 of the members 32 and 34. This RF heats and fuses the EVA layer 32a and 34a thereby creating a contents-retaining heat seal 44 indicated in solid lines.

In addition, as illustrated in Figure 9, electrodes E can be used to apply radio frequency energy to the region of the port 40 so as to bond the EVA layer 40a to the adjacent regions of the EVA layers 32a and 34a of the members 32 and 34. Hence, the container 30 as illustrated in Figure 8 includes the integrally bonded port 40 as well as a sealed body portion formed of the members 32 and 34.

Subsequently, the container 30 can be subjected to irradiation, on the order of 7.5 megarads and then can be filled with an autoclavable substance. A seal means 46 such as a high-density polyethylene membrane can be inserted into the port 40 in contact with the interior surface 40b. The seal means 46 can then be heat sealed to the high density, polyethylene member 40b closing the port 38. Alternately, the seal means 46 may be butt-welded to the port 38 by a hot-platen method.

The sealed and filled container can now be sterilized using steam sterilization. Alternately, dry heat sterilization can be used.

As a result of the cross-linking operation to which the members 32, 34 and the port 40 have been subjected, in combination with the high-density, polyethylene layer, the container 30 will be stable at temperatures on the order of 110-121°C or more during the sterilization process. Additionally, due to the exterior polyethylene layer, such as the layer 14 of the film 10, the container 30 will not become tacky and will not adhere to adjacent containers while being sterilized.

A further advantage of the container 30 is found in the fact that the additional structural integrity imparted to the container by the high-density, polyethylene layer minimizes shrinkage or distortion during the sterilization process due to internal stresses that might be formed in the EVA layer during the extrusion process. Additionally, the container 30 exhibits improved lower water vapor transmission characteristics and does not require an overpouch to avoid loss of water from aqueous contents during storage.

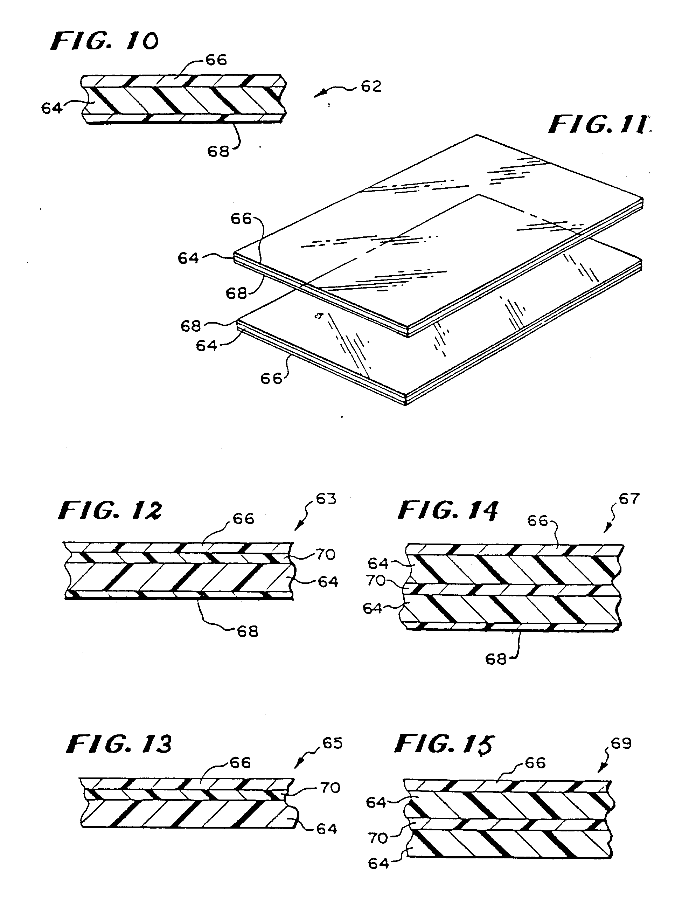

A multiple, three-layer composite film 62 which embodies the features of the invention is shown in Figs. 10 and 11. The first and second layers 64 and 66 correspond, respectively, to the EVA and polyethylene layers previously discussed.

In Fig. 10, a third or "skin layer" 68 is applied over the exposed EVA layer 64. This skin layer 68 can be a crystalline material such as polyethylene, polypropylene, or copolymers, blends, and alloys thereof. It can be 0.0001 to 0.001 inch 0.002 to 0.025 mm thick, with a preferred thickness of 0.0005 inch (0.008 mm).

If used to form a fluid retaining pouch or container of the types previously described, the skin layer 68 is inwardly oriented, as shown in Fig. 12. The multiple layer composite film 62 is especially useful in certain applications. In blood processing, for example, transfer sets are often formed with multiple, sterile, empty containers. The skin layer 68 keeps the EVA surfaces 64 from sticking together during sterilization. Thus, in addition to having non-tacking polyethylene exterior surfaces 66, such containers have non-tacking interior surfaces 68 as well.

Another application of the above-noted multiple layer composite film 62 is in containers whose contents might be readily absorbed by exposed surfaces of EVA material. For example, nitroglycerine is readily absorbed by EVA materials. However, such a substance could be stored in a container formed of a above-noted multiple layer composite film 62, as the skin layer 68 eliminates such absorption.

A multiple, four-layer composite film 63 which embodies the features of the invention is shown in Fig. 13. There, a fourth layer 70 is incorporated in the film between the layer of EVA material 64 and the thicker of the two layers of polyethylene, i.e. layer 66. The fourth layer 70 could be formed of poly(ethylene-vinyl alcohol), referred to as EVAL. This fourth layer 70 functions as a gas and vapor barrier layer. It can have a thickness in a range of 0.0001 to 0.005 inch (0.002 to 0.076 mm), preferably in a range of 0.005 to 0.002 inch (0.076 to 0.50 mm). In four layer films, both the skin layer 68 and the fourth layer 70 add further mechanical and thermal support to the EVA layer 64.

As shown in Fig. 13, the gas and vapor barrier layer 70 can be used in a three layer film 65 as well, positioned inbetween the EVA layer 64 and the polyethylene layer 66.

Another multiple layer composite film 67 which embodies the features of the invention is shown in Fig. 15. Here, the layer 70 is positioned within the EVA layer 64. In this arrangement, the layer 70 can be formed either of EVAL; poly(ethylene-vinyl alcohol); or poly (vinylidine chloride), referred to as PVDC. This layer 70 serves as a water and gas vapor barrier, as in the Figs. 13 and 14 embodiments.

As shown in Fig. 14, the composite film 67 can also include, in addition to the polyethylene layer 66, the skin layer 68 for the same purposes described with respect to the Figs. 11 to 13 embodiments. Alternately, as shown in Fig. 15, a composite film 69 can include the layer 70 positioned within the EVA layer 64 in conjunction with only the polyethylene layer 66.

The composite films 62, 63, 65, 67, and 69 can be formed by either lamination or coextrusion.

Containers formed of the above-noted multiple layer composite films can be readily heat sealed as discussed above. While the skin layer 68, if present, does not generate heat in the applied RF electrical field, at the above-noted thicknesses, it will absorb enough heat from the adjacent EVA layer and melt to form a suitable heat seal.

## Claims

1. A method of fabricating a heat-sterilisable substance-retaining container (30), comprising forming first and second film members (32,34), each having at least first and second integral layers (12,14,64,66), the first layer (12,64) being heat-sealable; positioning the first and second film members (32,34) in overlapping relationship, with corresponding first layers (12,64) of the film members being between the second layers (14,66) to form inner layers of the container; positioning a port (40) with an outer cylindrical surface (40a) between regions of the film members, and melting a peripheral region of the overlapping, first layers (12,64) using heat-sealing elements (E), so as to form the container by forming a substance-retaining seal (44) between the first layers thereby securing said port, the second layers staying cooler to prevent thinning or cut-through by the heat sealing elements (E), filling the container (30), and sterilising the filled container by exposure to heat, the first layers (12,64) being distortable at the sterilising temperature,

characterised in that:

the material of the first layers (12,64) is selected to be free, or essentially free, of leachable plasticisers and to be cross-linkable,

the material of the second layers (14,66) is selected not to be cross-linkable, as compared with the first layers (12,64), and to have a higher melting temperature so as not to be distortable at the sterilising temperature,

the material of the outer surface (40a) of the port (40) is selected to be sealed to the first layers (12,64) during the melting step and to be cross-linkable, and

the container (30) is irradiated, prior to the filling and sterilising-steps, to cross-link the first layers (12,64) and the outer surface (40a) of the port and provide resistance of the first layers (12,64) to distortion at the sterilising temperature.

2. A method as in Claim 1 wherein the dosage of irradiation is in a range of 5-10 megarads.

3. A method as in Claim 2 wherein the dosage of irradiation corresponds substantially to 7.5 megarads.

4. A method according to Claim 1, 2 or 3 and further including the step of sealing the port subsequent to filling the container.

5. A method as in any one of claims 1 to 4 including sterilizing the filled, sealed container with heated steam.

6. A method according to any preceding claim, wherein

said first layer (12,64) is formed of a selected, plasticizer-free, cross-linkable, poly-(ethylene vinyl acetate) material and said second layer (14,66) is formed of a high density material which is selected from polyethylene, polypropylene, or copolymers, blends, or alloys thereof along the exterior of said port, said first layer and the exterior of said port subsequently being cross-linked using a selected dose of irradiation; and

said container being filled with the selected contents, said port sealed with sealing means, and said sealed container and said contents being sterilizable as a sealed unit.

7. A method according to any preceding claim, wherein said port (40) has at least two integral layers with a first (40a) of said layers formed of a selected, plasticizer-free, cross-linkable, poly(ethylene vinyl acetate) material and with a second (40b) of said layers formed of a high density, substantially non-cross-linkable polyethylene material, said port being formed as a cylinder having said first layer (40a) positioned as the exterior layer.

8. A method as in any preceding claim wherein each said film member (32,34) is a coextruded film, or a laminated film.

9. A method as in any preceding Claim wherein said first layer (12,64) has a thickness in a range of 0.005-0.010 inch (0.13-0.25 mm) and wherein said second layer (14,66) has a thickness in a range of 0.002-0.004 inch (0.05 mm-0.10 mm).

10. A method as in Claim 9 wherein said first layer (12,64) has a thickness substantially equal to 0.006-0.008 inch (0.15-0.20 mm) or 0.007 to 0.008 inch (0.18-0.20 mm).

11. A method as in any preceding claim, wherein overlapping sections of said first layer (12,64) are melted and are fused together by selected radio frequency energy so as to form a heat seal prior to said container being exposed to irradiation for cross-linking.

12. A method as in Claim 7 wherein said poly-(ethylene vinyl acetate) contains 10 to 35 weight percent of vinyl acetate units.

13. A method as in any preceding claim wherein each said film member (32,34) has an integral additional layer (68) bonded to said first layer (64) with said third layer (68) positioned as the innermost layer of said container to prevent contact between the contents of said container and said first layer (64).

14. A method as in Claim 13 with said third layer (68) formed from material selected from polyethylene, polypropylene, or copolymers, blends, and alloys thereof.

15. A method as in Claim 14 wherein said third layer (68) has a thickness in a range of 0.0001 to 0.001 inch (0.002 to 0.025 mm)

**16.** A method as in any preceding claim wherein said film has a further layer (70) positioned within said first layer (64), or between said first and said second layers (64,66) to serve as an integral gas and water vapor barrier for said container.

**17.** A method as in Claim 16 wherein said further layer is formed of poly(ethylene vinyl alcohol) or poly(vinylidine chloride).

**18.** A method as in Claim 16 or 17 wherein said further layer has a thickness in a range of 0.0001 to 0.005 inch (0.002 to 0.127 mm).

**19.** A method as in Claim 1 wherein the second layer (14,66) serves as a water vapor barrier.

**20.** A method as in any preceding claim wherein said second layer (14,66) has a thickness substantially equal to 0.003 inch (0.08 mm).

**Patentansprüche**

**1.** Verfahren zum Herstellen eines wärmesterilisierbaren, zur Aufnahme einer Substanz dienenden Behälters (30), wobei das Verfahren aufweist: Formen eines ersten und eines zweiten flächigen Elements (32, 34), die jeweils wenigstens eine erste und eine zweite integrale Schicht (12, 14, 64, 66) haben, wobei die erste Schicht (12, 64) thermoschweißbar ist; Positionieren des ersten und des zweiten flächigen Elements (32, 34) in Überlappungsbeziehung, wobei entsprechende erste Schichten (12, 64) der flächigen Elemente zwischen den zweiten Schichten (14, 66) liegen, um innere Schichten des Behälters zu bilden; Positionieren eines Durchlasses (40) mit einer zylindrischen Außenfläche (40a) zwischen Zonen der flächigen Elemente und Anschmelzen einer Randzone der überlappten ersten Schichten (12, 64) unter Verwendung von Thermoschweißelementen (E), um den Behälter durch Formen einer der Rückhaltung einer Substanz dienenden Abdichtung (44) zwischen den ersten Schichten zu formen und dadurch den Durchlaß festzulegen, wobei die zweiten Schichten kühler bleiben, um ein Dünnerwerden oder Durchtrennen durch die Thermoschweißelemente (E) zu verhindern, Befüllen des Behälters (30) und Sterilisieren des befüllten Behälters durch Beaufschlagen mit Wärme, wobei die ersten Schichten (12, 64) bei der Sterilisierungstemperatur verformbar sind, dadurch gekennzeichnet, daß: das Material der ersten Schichten (12, 64) gewählt wird, um frei oder im wesentlichen frei von auslaugbaren Weichmachern und vernetzbar zu sein, das Material der zweiten Schichten (14, 66) gewählt wird, um im Vergleich mit den ersten Schichten (12, 64) nichtvernetzbar zu sein und eine höhere Schmelztemperatur zu haben, so daß es bei der Sterilisierungstemperatur nicht verformbar ist, das Material der Außenfläche (40a) des Durchlasses (40) gewählt wird, um während des Schmelzschritts mit den ersten Schichten (12, 64) verschweißbar und vernetzbar zu sein, und der Behälter (30) vor dem Befüll- und dem Sterilisierungsschritt bestrahlt wird, um die ersten Schichten (12, 64) und die Außenfläche (40) des Durchlasses zu vernetzen und den ersten Schichten (12, 64) Beständigkeit gegenüber Verformung bei der Sterilisierungstemperatur zu geben.

**2.** Verfahren nach Anspruch 1, wobei die Bestrahlungsdosis in einem Bereich von 5-10 Mrad liegt.

**3.** Verfahren nach Anspruch 2, wobei die Bestrahlungsdosis im wesentlichen 7,5 Mrad entspricht.

**4.** Verfahren nach Anspruch 1, 2 oder 3, das ferner den Schritt des dichten Verschließens des Durchlasses anschließend an das Befüllen des Behälters aufweist.

**5.** Verfahren nach einem der Ansprüche 1-4, das das Sterilisieren des befüllten, dicht verschlossenen Behälters mit Heißdampf aufweist.

**6.** Verfahren nach einem der vorhergehenden Ansprüche, wobei die erste Schicht (12, 64) aus einem ausgewählten weichmacherfreien vernetzbaren Poly(ethylen/Vinylacetat)-Material und die zweite Schicht (14, 66) aus einem Material hoher Dichte, das aus Polyethylen, Polypropylen oder Copolymeren, Gemischen oder Legierungen davon ausgewählt ist, geformt ist.

**7.** Verfahren nach einem der vorhergehenden Ansprüche, wobei der Durchlaß (40) wenigstens zwei integrale Schichten hat, wobei eine erste (40a) dieser Schichten aus einem ausgewählten weichmacherfreien vernetzbaren Poly-(ethylen/Vinylacetat)Material geformt ist und eine zweite (40b) dieser Schichten aus einem hochdichten, im wesentlichen nichtvernetzbaren Polyethylenmaterial geformt ist, wobei der Durchlaß als ein Zylinder geformt ist, auf dem die erste Schicht (40a) als die Außenschicht positioniert ist.

8. Verfahren nach einem der vorhergehenden Ansprüche, wobei jedes flächige Element (32, 34) eine koextrudierte Folie oder eine Verbundfolie ist.

9. Verfahren nach einem der vorhergehenden Ansprüche, wobei die erste Schicht (12, 64) eine Dicke in einem Bereich von 0,13-0,25 mm (0,005-0,010 inch) hat und wobei die zweite Schicht (14, 66) eine Dicke in einem Bereich von 0,05-0,10 mm (0,002-0,004 inch) hat.

10. Verfahren nach Anspruch 9, wobei die erste Schicht (12, 64) eine Dicke hat, die im wesentlichen gleich 0,15-0,20 mm (0,006-0,008 inch) oder 0,18-0,20 mm (0,007-0,008 inch) ist.

11. Verfahren nach einem der vorhergehenden Ansprüche, wobei überlappte Abschnitte der ersten Schicht (12, 64) durch ausgewählte Hochfrequenzenergie angeschmolzen und miteinander verschmolzen werden, um eine Thermoschweißverbindung zu bilden, bevor der Behälter einer Vernetzungsbestrahlung ausgesetzt wird.

12. Verfahren nach Anspruch 7, wobei das Poly(ethylen/Vinylacetat) 10-30 Gew.-% Vinylacetat-Einheiten enthält.

13. Verfahren nach einem der vorhergehenden Ansprüche, wobei jedes flächige Element (32, 34) eine auf die erste Schicht (64) bondierte integrale zusätzliche Schicht (68) hat, wobei diese dritte Schicht (68) als die innerste Schicht des Behälters angeordnet ist, um einen Kontakt zwischen dem Inhalt des Behälters und der ersten Schicht (64) zu verhindern.

14. Verfahren nach Anspruch 13, wobei die dritte Schicht (68) aus Material geformt ist, das aus Polyethylen, Polypropylen oder Copolymeren, Gemischen und Legierungen davon ausgewählt ist.

15. Verfahren nach Anspruch 14, wobei die dritte Schicht (68) eine Dicke in einem Bereich von 0,002-0,025 mm (0,0001-0,001 inch) hat.

16. Verfahren nach einem der vorhergehenden Ansprüche, wobei die Folie eine weitere Schicht (70) hat, die innerhalb der ersten Schicht (64) oder zwischen der ersten und der zweiten Schicht (64, 66) positioniert ist, um als eine integrale Gas- und Wasserdampfsperre für den Behälter zu dienen.

17. Verfahren nach Anspruch 16, wobei die weitere Schicht aus Poly(ethylen/Vinylalkohol) oder Poly(vinylidenchlorid) geformt ist.

18. Verfahren nach Anspruch 16 oder 17, wobei die weitere Schicht eine Dicke in einem Bereich von 0,002-0,127 mm (0,0001-0,005 inch) hat.

19. Verfahren nach Anspruch 1, wobei die zweite Schicht (14, 66) als eine Wasserdampfsperre dient.

20. Verfahren nach einem der vorhergehenden Ansprüche, wobei die zweite Schicht (14, 66) eine Dicke von im wesentlichen gleich 0,08 mm (0,003 inch) hat.

**Revendications**

1. Procédé de fabrication d'un récipient (30) retenant les substances et stérilisable par la chaleur, consistant à former un premieret un second éléments de film (32, 34), comportant chacun au moins une première et une seconde couches (12, 14, 64, 66) formant bloc, la première couche (12, 64) étant thermoscellable, à positionner ces premier et second éléments de film (32, 34) suivant une disposition relative superposée, avec les premières couches correspondantes (12, 64) des éléments de film situées entre les secondes couches (14, 66) de façon à former des couches intérieures du récipient, à positionner un orifice (40), comportant une surface cylindrique extérieure (40a) entre des zones des éléments de film, à faire fondre une zone périphérique des premières couches (12, 64), superposées, en utilisant des éléments de thermoscellement (E), de façon à former le récipient en réalisant un joint (44), retenant les substances, entre les premières couches, ce qui fixe ainsi l'orifice, les secondes couches restant plus froides afin d'empêcher un amincissement ou une traversée par les éléments de thermoscellage (E), à remplir le récipient (30) et à stériliser le récipient rempli par exposition à la chaleur, les premières couches (12, 64) pouvant être l'objet de déformations à la température de stérilisation,

caractérisée en ce que :

la matière des premières couches (12, 64) est choisie de façon à être exempte, ou pratiquement exempte, de plastifiants pouvant être éliminés par lixiviation et de façon à être réticulable,

la matière des secondes couches (14, 66) est choisie de façon à ne pas être réticulable, par comparaison avec les premières couches (12, 64), et de façon à posséder une température plus élevée de fusion, de façon à ne pas pouvoir faire l'objet de déformations à la température de stérilisation,

la matière de la surface extérieure (40a) de l'orifice (40) est choisie de façon à être scellée sur les premières couches (12, 64) pendant l'opération de fusion et de façon à être réticulable et

le récipient (30) est soumis à un rayonnement, avant les opérations de remplissage et de stérilisation, afin de réticuler les premières couches (12, 64) et la surface extérieure (40a) de l'orifice et à conférer aux premières couches (12, 64) de la résistance aux déformations à la température de stérilisation.

2. Procédé suivant la revendication 1, selon lequel la dose d'irradiation est comprise entre 5 et 10 mégarads.

3. Procédé suivant la revendication 2, selon lequel la dose d'irradiation est pratiquement égale à 7,5 mégarads.

4. Procédé suivant la revendication 1, 2 ou 3, comportant en outre l'opération consistant à sceller l'orifice à la suite du remplissage du récipient.

5. Procédé suivant l'une quelconque des revendications 1 à 4, comprenant l'opération consistant à stériliser le récipient, rempli et scellé, au moyen de vapeur d'eau chauffée.

6. Procédé suivant l'une quelconque des revendications précédentes, selon lequel la première couche (12, 64) est réalisée en une matière choisie, formée de poly(éthylène-acétate de vinyle), réticulable et exempte de plastifiant, et la seconde couche (14, 66) est réalisée en une matière à haute densité qui est choisie parmi un polyéthylène, un polypropylène ou des copolymères, des mélanges ou des combinaisons de ceux-ci.

7. Procédé suivant l'une quelconque des revendications précédentes, selon lequel l'orifice (40) comprend au moins deux couches formant bloc, une première (40a) de ces couches étant réalisée en une matière choisie, formée de poly(éthylène-acétate de vinyle), réticulable et exempte de plastifiant, et la seconde (40b) de ces couches étant réalisée en une matière à haute densité, formée de polyéthylène pratiquement non réticulable, l'orifice se présentant sous la forme d'un cylindre dont la première couche (40a) est placée de façon à constituer la couche extérieure.

8. Procédé suivant l'une quelconque des revendications précédentes, selon lequel chaque élément de film (32,34) est constitué d'un film coextrudé ou d'un film obtenu par superposition stratifiée.

9. Procédé suivant l'une quelconque des revendications précédentes, selon lequel la première couche (12, 64) a une épaisseur comprise entre 0,005 et 0,010 pouce (0,13 et 0,25 mm) et la seconde couche (15, 66) a une épaisseur comprise entre 0,002 et 0,004 pouce (0,05 mm et 0,10 mm).

10. Procédé suivant la revendication 9, selon lequel la première couche (12, 64) a une épaisseur pratiquement égale à 0,006-0,008 pouce (0,15-0,20 mm) ou à 0,007-0,008 pouce(0,18-0,20 mm).

11. Procédé suivant l'une quelconque des revendications précédentes, selon lequel on fait fondre et fusionner entre elles des sections superposées de la première couche (12, 64) au moyen d'une énergie haute fréquence choisie, de façon à former un joint thermoscellé avant que le récipient soit soumis à un rayonnement en vue de sa réticulation.

12. Procédé suivant la revendication 7, selon lequel le poly(éthylène-acétate de vinyle) contient de 10 à 35 % en poids d'unités d'acétate de vinyle.

13. Procédé suivant l'une quelconque des revendications précédentes, selon lequel chaque élément de film (32, 34) comporte une couche supplémentaire (68) formant bloc qui est collée à la première couche (64), la troisième couche (68) étant placée de façon à constituer la couche la plus intérieure du récipient afin d'empêcher un contact entre le contenu du récipient et la première couche (64).

14. Procédé suivant la revendication 13, selon lequel la troisième couche (68) est réalisée en une matière choisie parmi un polyéthylène, un polypropylène ou des copolymères, des mélanges ou des combinaisons de ceux-ci.

15. Procédé suivant la revendication 14, selon lequel la troisième couche (68) a une épaisseur comprise entre 0,0001 et 0,001 pouce (0,002 et 0,025 mm).

**16.** Procédé suivant l'une quelconque des revendications précédentes, selon lequel le film comporte une autre couche (70) placée au milieu de la première couche (64) ou entre la première et la seconde couches (64,66), de façon à servir d'écran aux gaz et à la vapeur d'eau, formant bloc, pour le récipient.

**17.** Procédé suivant la revendication 16, selon lequel l'autre couche est réalisée en un poly-(éthylène-alcool vinylique) ou un polychlorure de vinylidène.

**18.** Procédé suivant la revendication 16 ou 17, selon lequel l'autre couche a une épaisseur comprise entre 0,0001 et 0,005 pouce (0,002 et 0,127 mm).

**19.** Procédé suivant la revendication 1, selon lequel la seconde couche (14, 66) sert d'écran à la vapeur d'eau.

**20.** Procédé suivant l'une quelconque des revendications précédentes, selon lequel la seconde couche (14, 66) a une épaisseur pratiquement égale à 0,003 pouce (0,08 mm).

**FIG. 1**

**FIG. 2**

**FIG. 3**

**FIG. 4**

**FIG. 5**

*FIG. 6*

*FIG. 7*

*FIG. 8*

*FIG. 9*

**FIG. 10**

66

64

68

62

**FIG. 11**

66

64

68

68

64

66

**FIG. 12**

66

63

70

64

68

**FIG. 14**

66

67

64

70

64

68

**FIG. 13**

66

65

70

64

**FIG. 15**

66

69

64

70

64